# EUROPEAN PATENT SPECIFICATION

(11) **EP 2 297 122 B1**
(45) Date of publication and mention of the grant of the patent: **21.05.2014**
(21) Application number: 09761710.4
(22) Date of filing: 09.06.2009
(51) Int. Cl.: C07D 295/185

(54) **NEW PROCESS FOR THE PREPARATION OF LEVOCETIRIZINE AND INTERMEDIATES THEREOF**
NEUES VERFAHREN ZUR HERSTELLUNG VON LEVOCETIRIZIN UND ZWISCHENPRODUKTEN DAVON
NOUVEAU PROCÉDÉ POUR LA PRÉPARATION DE LA LÉVOCÉTIRIZINE ET DE SES INTERMÉDIAIRES

(30) Priority: 11.06.2008 EP 08158058
(43) Date of publication of application: 23.03.2011
(73) Proprietor: KRKA, tovarna zdravil, d.d., Novo mesto, 8501 Novo mesto (SI)
(72) Inventor: TIHI, Jaroslav, 8000 Novo mesto (SI); PECAVAR, Anica, Novo mesto 8000 (SI); ZUPET, Rok, 1000 Ljubljana (SI); RZEN, Janez, 1000 Ljubljana Zalog (SI); STROPNIK, Tadej, 8000 Novo Mesto (SI); HVALEC, Miran, 8000 Novo mesto (SI); KLOBCAR, Andrej, 8000 Novo mesto (SI); BENKIC, Primoz, 1000 Ljubljana (SI); SMODIS, Janez, 8000 Novo mesto (SI); KRASOVEC, Dusan, 8296 Krmelj (SI)
(74) Representative: UEXKÜLL & STOLBERG
(86) International application number: PCT/EP2009/057081
(87) International publication number: WO 2009/150147

(56) References cited:
- WO-A-01/29016
- WO-A-2008/110586
- GB-A- 2 225 321

## Description

### Field of the invention

The present invention describes a novel process for the preparation of levocetirizine and intermediates thereof, and its pharmaceutically acceptable salts and esters.

### Background of the invention

Levorotatory [2-[4-[(4-chlorophenyl)phenylmethyl]-1-piperazin-yl]ethoxy]acetic acid, also known by the generic name of levocetirizine, has proven useful as a therapeutic agent for the treatment of allergic disease.

Levocetirizine and its salts including its dihydrochloride are known and are effective in the treatment of allergies, including but not limited to, chronic and acute allergic rhinitis, allergic conjunctivitis, pruritus, urticaria and the like. Levocetirizine belongs to the second generation of H1 histamine receptor antagonists, which are believed to offer significant advantages over first generation compounds. Studies have shown that levocetirizine provides safe and effective symptomatic relief of seasonal allergies. Levocetirizine is used also for treating chronic idiopathic urticaria.

GB 2,225,321 describes a process for the preparation of cetirizine in the levorotatory form, dextrorotatory form or a mixture thereof comprising the hydrolysis of enantiomerically pure [2-[4-[(4-chlorophenyl)phenylmethyl]-1-piperazinyl]ethoxy]aceto-nitrile. Hydrolysis takes place in aqueous, alcoholic or aqueous-alcoholic medium by a base or by an acid; the acid thus obtained is converted to its dihydrochloride. Optically active starting material 1-[(4-chlorophenyl)phenylmethyl]piperazine is obtained by resolution of the corresponding racemic compound, preferably by conversion to its diastereoisomeric salt with tartaric acid. The yield of resolution is rather low, namely only 12.7%. The obtained optically active intermediate is further converted with chloroethoxyacetonitrile in 69% yield.

EP 0 617 028 and EP 0 955 295 disclose a process for the preparation of optically active 1-[(4-chlorophenyl)phenylmethyl]piperazine and its conversion to cetirizine in the levorotatory form or dextrorotatory form or to derivative thereof. The process for the preparation is shown in the following scheme:

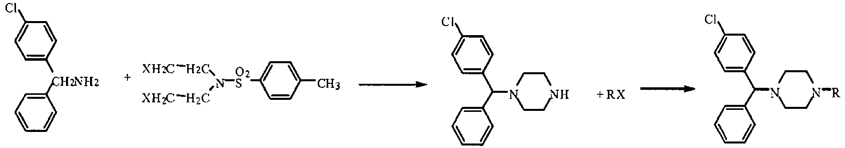

The drawback of the disclosed reaction is that it requires protection of N,N-bis(2-haloethyl)amine, and consequently deprotection of the intermediate obtained.

Preparation of cetirizine in its levorotatory form proceeds in most known syntheses from enantiomerically pure 1-[(4-chlorophenyl)phenylmethyl]piperazine. Consequently it appears to be very desirable to provide new routes to prepare the enantiomers thereof with improved optical purity and good yields.

Polymorphic form I of crystalline levorotatory dihydrochloride salt of cetirizine and amorphous form thereof are disclosed in WO 2004/050647 and WO 2004/065360. Crystalline form is prepared by crystallization from ketone-containing solvent, such as acetone, methyl ethyl ketone, dimethylketone, 2-pentanone and mixtures thereof. Amorphous form was prepared by solvent evaporation.

There still exists a need for an efficient synthesis of levocetirizine, new intermediates used in the process, suitable for large-scale production.

### Brief Description of the Drawings

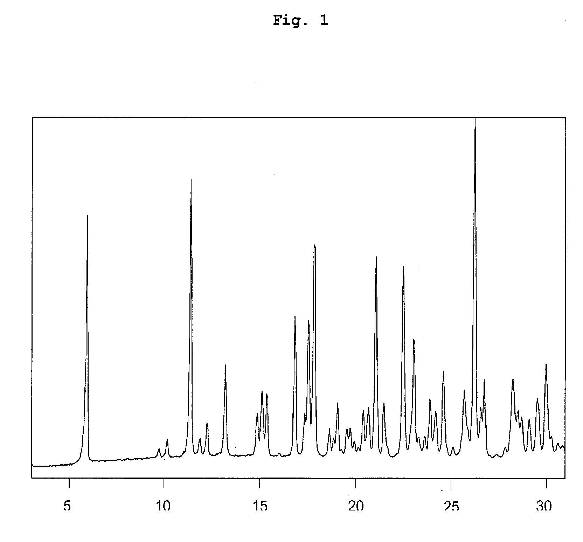

Fig. 1 represents an X-ray powder diffraction pattern of levocetirizine dihydrochloride solvate with acetic acid. This and all other X-ray powder diffraction patterns reported herein were recorded with a Phillips PW3040/60 X'Pert PRO diffractometer; CuKα radiation 1,541874 Å, if not indicated otherwise.

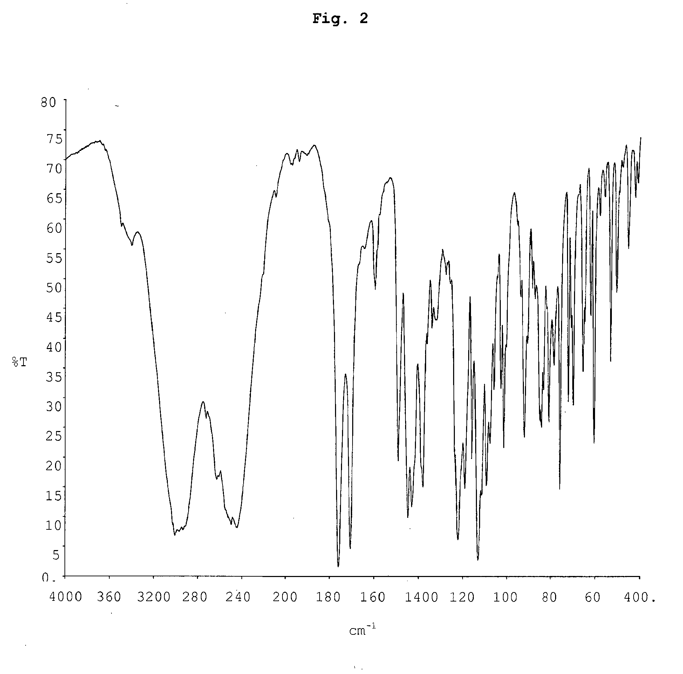

Fig. 2 represents an FT-IR spectrum of levocetirizine dihydrochloride solvate with acetic acid. This and all other FT-IR spectra reported herein were recorded on a FT-IR System SPECTRUM 1000 Perkin-Elmer [4000-400 cm-1, Resolution 4 cm-1, KBr tbl.], if not indicated otherwise.

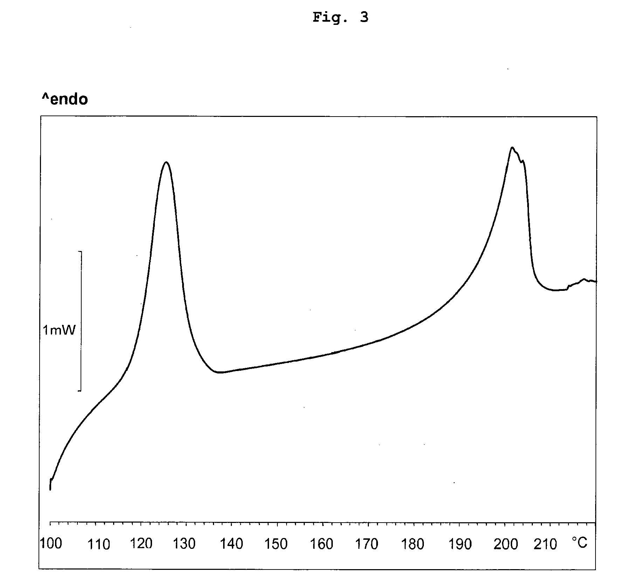

Fig. 3 represents a DSC thermogram of levocetirizine dihydrochloride solvate with acetic acid. This and all other DSC thermograms reported herein were recorded on a DSC 822 Mettler Toledo scanning calorimeter [N2, 10°C/min], if not indicated otherwise.

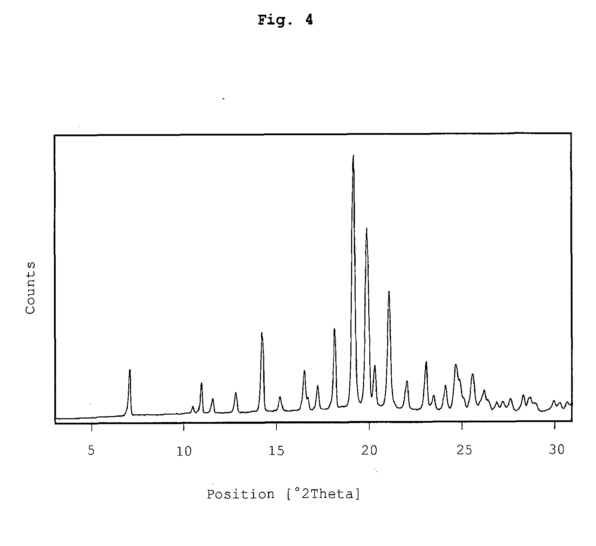

Fig. 4 represents an X-ray powder diffraction pattern of R-2-(2-(4-((4-chlorophenyl)(phenyl)methyl)piperazin-1-yl-2-oxoethoxy) acetic acid (ketocetirizine).

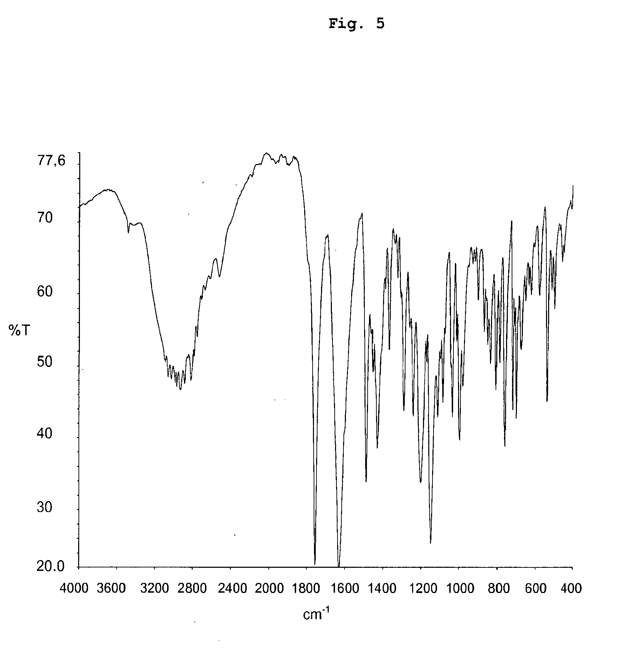

Fig. 5 represents an FT-IR spectrum of R-2-(2-(4-((4-chlorophenyl)(phenyl)methyl)piperazin-1-yl-2-oxoethoxy)acetic acid (ketocetirizine).

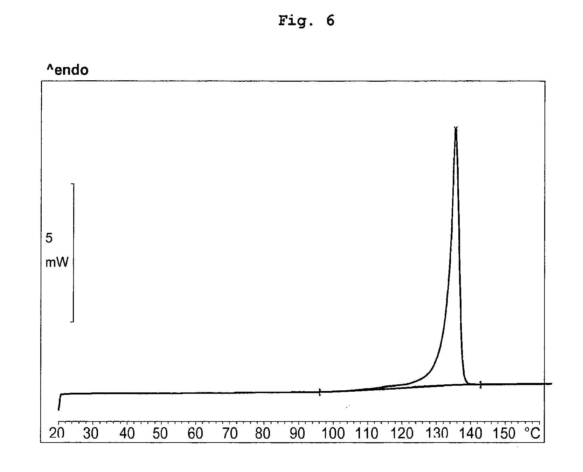

Fig. 6 represents a DSC thermogram of R-2-(2-(4-((4-chlorophenyl)(phenyl)methyl)piperazin-1-yl-2-oxoethoxy)acetic acid (ketocetirizine).

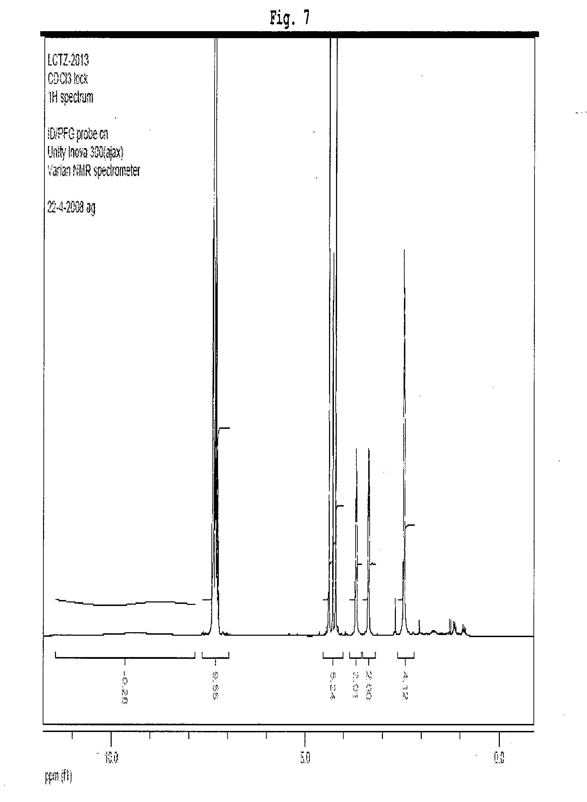

Fig. 7 represents a NMR spectrum of R-2-(2-(4-((4-chlorophenyl)(phenyl)methyl)piperazin-1-y1-2-oxoethoxy)acetic acid (ketocetirizine). This and all other NMR spectra reported herein were recorded on Varian Inova 300 MHz spectrometer, if not indicated otherwise.

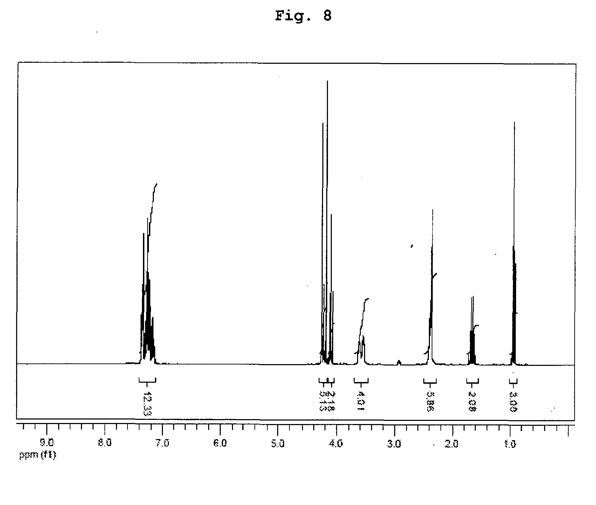

Fig. 8 represents a NMR spectrum of propyl R-2-(2-(4-((4-chlorophenyl)phenylmethyl)piperazin-1-yl)-2-oxoethoxy)acetate (propyl ester of ketocetirizine).

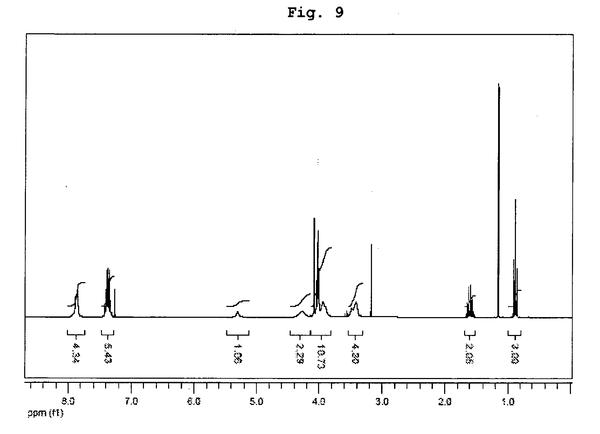

Fig. 9 represents a NMR spectrum of propyl ester of levocetirizine dihydrochloride.

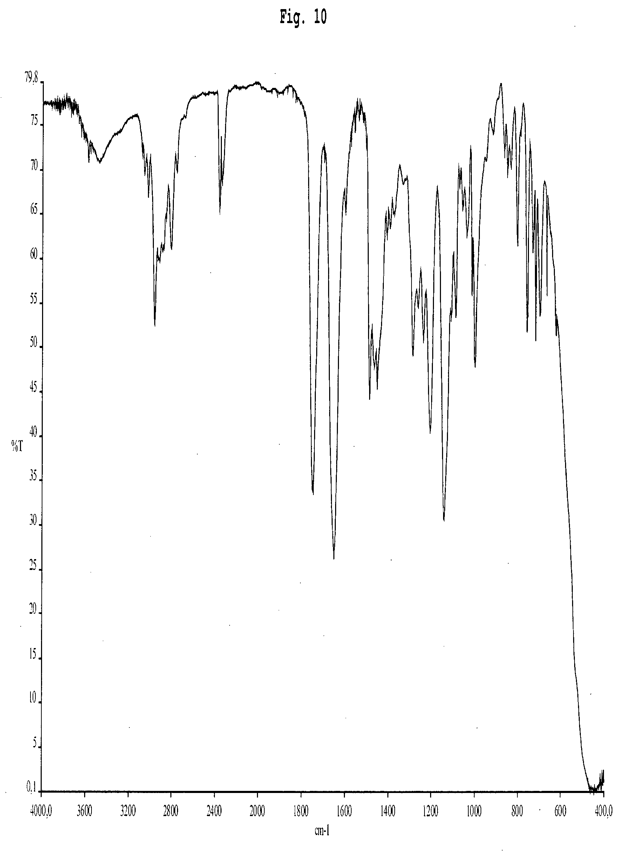

Fig. 10 represents a FT-IR spectrum of propyl R-2-(2-(4-((4-chlorophenyl)phenylmethyl)piperazin-1-yl)-2-oxoethoxy)acetate (propyl ester of ketocetirizine).

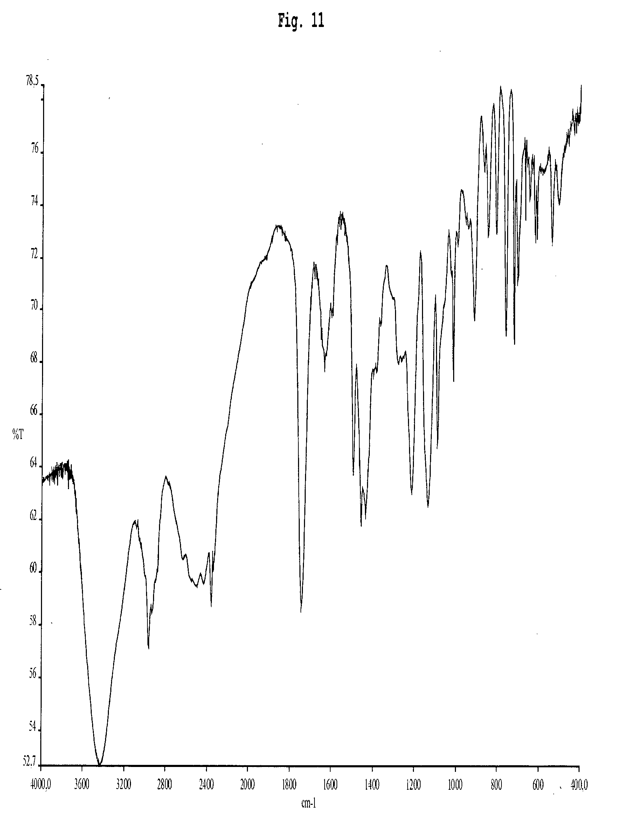

Fig. 11 represents a FT-IR spectrum of propyl ester of levocetirizine dihydrochloride.

### Description of the invention

The present invention provides a new efficient synthesis of levocetirizine and pharmaceutically acceptable salts thereof and new intermediates used in that process.

According to a first aspect, the present invention relates to a process for production of levocetirizine, comprising the following steps:
b) reaction of ketocetirizine of the formula IIIb 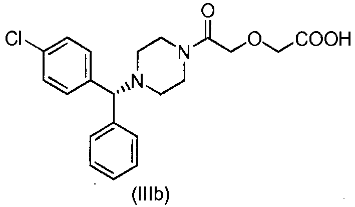 to a ketocetirizine ester of the formula IV or a salt thereof 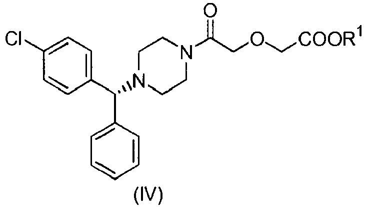 wherein R¹ is selected from alkyl groups, aryl groups, aryl substituted alkyl groups and organosilyl groups; and
c) conversion of the ketocetirizine ester IV or the salt thereof to levocetirizine or a pharmaceutically acceptable salt thereof.

In formula IV above R¹ is an alkyl group, an aryl group, an aryl substituted alkyl group or an organosilyl group. The alkyl group is typically a straight-chain or branched-chain alkyl group, preferably a lower alkyl group having 1 to 6 carbon atoms such as methyl, ethyl, isopropyl, n-propyl, and butyl (including t-butyl and n-butyl). More preferred are methyl and n-propyl. The term "aryl group" refers to substituted or unsubstituted aryl groups. The alkyl group is typically an aryl substituted straight-chain or branched-chain akyl group, such as benzyl or triphenylmethyl. The organosilyl group is typically a triorganosilyl group, preferably SiR⁴₃, wherein R⁴ is alkyl, or aryl including alkyl substituted aryl and halogen substituted aryl, more preferably straight-chain or branched-chain C₁ to C₆ alkyl; and most preferably methyl, propyl and isopropyl.

The term "ketocetirizine ester" as used herein for the compound of formula IV thus includes both esters with alcohols as well as silyl esters.

In step (b) the ketocetirizine ester IV can be prepared by reacting ketocetirizine of formula IIIb with an alcohol R¹OH, wherein R¹ is an organic group as defined above including preferred embodiments, or with an organo halosilane, preferably a triorgano chlorosilane R⁴₃SiCl, wherein R⁴ is defined as above, and more preferably trimethyl chlorosilane Me₃SiCl. The reaction is preferably carried out in the presence of mineral acid like sulphuric acid or hydrochloric acid.

The esterification with an alcohol may be conducted in such a way that the ketocetirizine is dissolved in the alcohol and then acid catalysis is performed. Examples of suitable acids that may be used include inorganic acids such as hydrogen chloride, phosphoric acid, and sulphuric acid; and organic acids such as p-toluenesulphonic acid, methanesulphonic acid, trifluoroacetic acid, and acetic acid. The reaction can be performed in alcohol as a solvent, preferably n-propanol for the preparation of n-propyl ester; other solvents such as aprotic solvents can also be used, for example ethers, aliphatic and aromatic hydrocarbons.

The reaction of the ketocetirizine with the organo halosilane for the preparation of silyl esters can also be performed in a suitable solvent. Exemplary solvents include those that are mentioned above in the context of the esterification with an alcohol.

Step (b) can be performed at room temperature in order to avoid side reactions. Lower temperatures such as down to 0°C, typically from about 10 to 0°C can also be used.

In one embodiment of the present process the ketocetirizine ester IV is prepared via the ketocetirizine chloride of formula VI

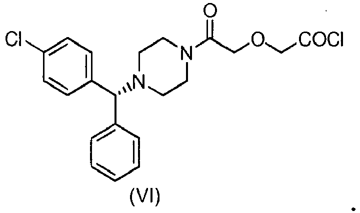

The ketocetirizine chloride VI can easily be reacted with a suitable alcohol or with an organo silanol to obtain the desired ester. The chloride can be prepared in a suitable solvent like THF, ether, or toluene, in the presence of a suitable chlorinating agent such as oxalyl dichloride, PCl₅, POCl₃, or SOCl₂, with oxalyl dichloride being preferred. It has been found that it is not mandatory to isolate the ketocetirizine chloride VI before it is further reacted. Thus, in one embodiment of the invention the ketocetirizine chloride is formed in-situ in step (b).

In step (c) the ketocetirizine ester IV or its salt is converted to levocetirizine or a pharmaceutically acceptable salt thereof. Typically the step (c) comprises:
i) reduction of the ketocetirizine ester of the formula IV 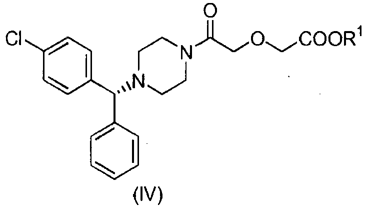 wherein R¹ is defined as in claim 1,
   with a selective reducing agent to obtain an levocetirizine ester of the formula V 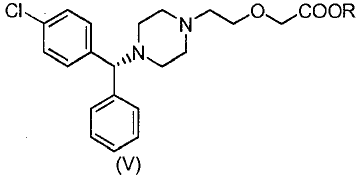 wherein R¹ is as defined above;
ii) conversion of the levocetirizine ester V to levocetirizine, and
iii) optionally conversion of levocetirizine to the pharmaceutically acceptable salt thereof.

Selective reducing agents used in step (i) may be selected from the group consisting of NaBH₄ optionally in the presence of carboxylic acids such as acetic acid, trifluoroacetic acid, formic acid or in the presence of sulphonic acids; NaBH₃CN optionally in the presence of carboxylic acids such as acetic acid, propanoic acid, trifluoroacetic acid; NaBH₃0COR₃ or NaBH(COOR³)₃, wherein R³ is methyl, trifluoromethyl and the like; boranes such as borane-solvent complexes, wherein the solvent is selected from tetrahydrofuran (H₃B-THF), dimethyl sulphide (H₃B-SMe₂, BMS), diethyl ether (H₃B-diethylether) ; R⁵₃OBF₄/NaBH₄, wherein R⁵ is methyl, ethyl, propyl and the like. Preferred selective reducing agents are the borane-solvent complexes, especially H₃B-THF and H₃B-SMe₂.

The suitable solvent used in the reduction phase is an inert organic solvent which may be selected from the group consisting of ethers such as dioxane, tetrahydrofuran, t-butylmethylether, diethylether, diisopropylether, 2-methyltetrahydrofuran; carboxylic acids such as acetic acid; halogenated hydrocarbons; aromatic hydrocarbons such as toluene. The preferred solvents are THF, toluene and dioxane.

Typically, the ratio of the reducing agent to the ketocetirizine ester IV is from 6:1 to 1:1, preferably 3:1 to 1:1, more preferably 1.75:1 to 1:1, most preferably 1.2:1 to 1:1.

The reaction is typically carried out in that way that a solution of ketocetirizine IV in a solvent is added to a borane solution at a temperature from 0°C to room temperature. The resulting mixture is heated to a temperature of from room temperature to the boiling point of reaction mixture and maintained there for 0.5 hours to several hours to drive the reaction essentially to completion.

The conversion of the levocetirizine ester V to levocetirizine in step (ii) is a hydrolysis reaction which can be performed under basic or acidic conditions. Basic hydrolysis of the levocetirizine ester, e.g. the n-propyl ester, is typically performed in water in the presence of an alkali metal hydroxide such as sodium or potassium hydroxide. Optionally, some amount of a C₁ to C₅ alcohol can be added. The product obtained is levocetirizine in the form of an alkaline metal salt. In one embodiment the hydrolysis of the levocetirizine ester V is carried out in a two phase system, for example toluene / water comprising alkaline metal hydroxide, preferably at elevated temperature.

After the reaction is completed the product is isolated and purified by conventional means such as extraction and crystallization. Purification by extraction preferably comprises several steps. Preferably, in the first extraction step alkaline extraction at pH 7 to 13, more preferably 7 to 10.5 is used. For this purpose the reaction mixture is mixed with water and then extracted with a halogenated or non-halogenated water-immiscible solvent such as ethyl acetate, methylene chloride (dichloromethane) or toluene. In the second extraction the pH is preferably adjusted to pH 4-5, more preferably to 4.2-4.8. This step can be performed by using the same solvent system as in the first step, i.e. water / halogenated or nonhalogenated water immiscible solvent such as ethyl acetate, methylene chloride or toluene.

The alkaline salt of levocetirizine which is the direct product of the basic hydrolysis is transformed to the free levocetirizine acid by acidification. The acidic extraction conditions used in the second extraction step above result in the formation of the free levocetirizine acid. The levocetirizine may be obtained as an residue by evaporation of the extraction solvent.

In case of acidic hydrolysis, the levocetirizine ester V is heated in the presence of an inorganic acid, such as hydrochloric acid, preferably in an aqueous medium, at a temperature between 60°C and the reflux temperature of the reaction mixture. Levocetirizine acid is then extracted from the reaction mixture by means of an organic solvent such as dichloromethane, toluene, ethylacetate, preferably from dichloromethane.

In optional step (iii) the free levocetirizine can be converted to the levocetirizine dihydrochloride e.g. by dissolving it in a solvent and introducing HCl in gaseous form into the reaction mixture and/or by introducing a solution of HCl gas in a solvent, until the pH reaches a value between 0.5 to 3, preferably 0.5 to 1. The solvent is typically a ketone solvent which may be selected from the group of acetone, methyl ethyl ketone, diisobutyl ketone, and dissopropyl ketone, preferably acetone. Other suitable solvents include alcohols, aromatic hydrocarbons, esters, and ethers, preferably toluene, ethyl acetate, propyl acetate, isopropyl acetate, and butyl acetate. The obtained salt can exist in polymorphic form disclosed in WO2004/050647 or IPCOM 000146553D.

Step(b) and step (c) of the process according to the invention may optionally be conducted without intermediate isolation of the ketocetirizine ester IV, i.e. as a one-pot reaction.

The present process optionally comprises as step (a) the reaction of an intermediate of the formula II 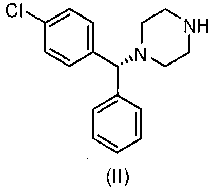 to ketocetirizine of formula IIIb prior to step (b). Typically, the intermediate of formula II is reacted with diglycolic acid anhydride of formula 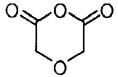 in an aprotic solvent to obtain ketocetirizine of formula IIIb. More specifically, an excess or substantially equimolar amounts of diglycolic acid anhydride may be employed, preferably 1.5 molar excess, most preferably 1.1 molar excess is employed. The reaction may be carried out in an organic solvent, water or mixture thereof. In case water is used as a solvent, the process can be carried out in the presence of a phase transfer catalyst. Suitable organic solvents include polar or non-polar organic solvents such as dimethylformamide, dimethylsulphoxide, acetonitrile; halocarbons such as chloroform, dichloromethane, carbon tetrachloride, 1,2-dichloroethane, and the like; ethers, such as diethyl ether, dioxane, tetrahydrofuran, 1,3-dimethoxyethane and the like, aromatic hydrocarbons such as benzene, toluene, xylene. The reaction may be carried out in the absence of a solvent.

The reaction can also be performed in the presence of tetrabutylammonium chloride, tetrabutylammmonium bromide, tetrabutylammonium cyanide, tetrabutylammonium fluoride, tetrabutylammonium iodide, tetrabutylammonium hydroxide, tetrabutylphosphonium chloride, tricaprylylmethylammonium chloride, tetraethylammonium chloride, tetramethylammonium bromide, trioctylethylphosphonium bromide, trioctylmethylammonium chloride, trioctylpropylammonium chloride, tetrapropylammonium bromide, tetraphenylarsonium chloride, tetraphenylphosphonium bromide, tetraphenylphosphonium chloride, benzyltrimethylammonium hydroxide, 18-crown-6, dibenzo-18-crown-6, dicyclohexyl-18-crown-6 or mixtures thereof. Preferably, tetra-n-butylammonium bromide or tetra-n-butylammonium iodide is used.

The temperature of the reaction may be from about 0°C to the boiling point of the reaction mixture, with the range from about 60°C to the boiling point of the reaction mixture being preferred. The reaction time sufficient to substantially complete the reaction is generally from 1 hour to 24 hours.

In carrying out the reaction the reactants are typically intimately admixed, preferably by adding portionwise while stirring a solution of diglycolic acid anhydride in a solvent to a solution of the amine intermediate of formula II also in a solvent, and the mixing is continued with or without the application of heat for a time sufficient to complete the reaction with the formation of desired product ketocetirizine in the reaction mixture. The product may be recovered or purified by conventional procedures, such as extraction, chromatography or evaporating the solvent in whole or in part by conventional means and recovering the solid or oil which separates out or remains as residue.

R-2-(2-(4-((4-chlorophenyl)(phenyl)methyl)piperazin-1-yl-2-oxoethoxy)acetic acid (ketocetirizine) is characterized by an X-ray powder diffractogram having peaks at about

| **No.** | **Pos. [°2Th.]** | **d-spacing [A]** | **Rel. Int. [%]** |
|---|---|---|---|
| | | | |
| 1 | 7,1 | 12,48 | 19 |
| 2 | 14,2 | 6,22 | 32 |
| 3 | 16,5 | 5,37 | 17 |
| 4 | 18,2 | 4,88 | 33 |
| 5 | 19,2 | 4,63 | 100 |
| 6 | 19,9 | 4,46 | 71 |
| 7 | 21,1 | 4,21 | 46 |
| 8 | 23,1 | 3,86 | 20 |

The amine intermediate compound of formula II used as a starting compound in step (a) according to present invention can be prepared in accordance with the following process or any other known process such as disclosed in GB 2,225,321, EP 0 617 028, IN 501/MUM/04.

The amine intermediate compound of formula II, (-)-1-[(4-chlorophenyl)phenylmethyl]piperazine, can be prepared in an efficient way by reaction of R-(-)-4-chlorobenzhydryamine and bis(2-haloethyl)amine hydrochloride. It was surprisingly found that no racemisation occurs during that reaction.

The reaction is shown in the following reaction scheme 1

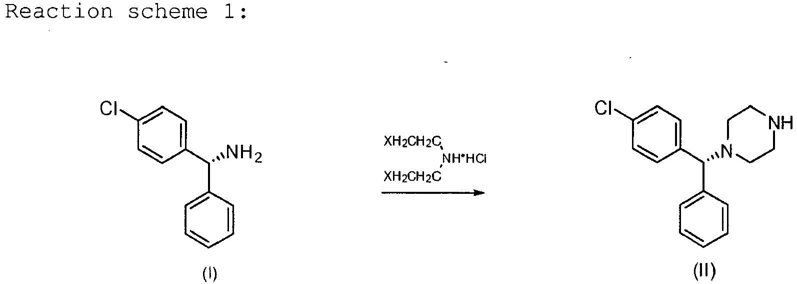

Bis(2-haloethyl)amine hydrochloride can be used, wherein X is a chlorine, bromine or iodine atom.

The reaction is typically carried out in the presence of an organic base which acts as a solvent and hydrogen chloride scavenger at the same time. Organic bases can be selected from the group consisting of primary, secondary, tertiary alkylamines such as n-ethyldiisopropylamine, triethylamine, diethylamine, butylcyclohexylamine, diisopropylamine, dibutylamine, or heterocyclic amines, such as, pyrrolidine, piperidine, or an alkyl substituted derivative thereof or a mixture thereof. The mixture of various bases can be mixed in any ratio, preferably mixture of n-ethyldiisopropylamine and diethylamine is used in the volume ratio from 1:1 to 1:0.01, most preferably 1: 0.08.

The reaction is generally carried out at a temperature of from 50°C to the reflux of reaction mixture, preferably from 80°C to the reflux of reaction mixture. The reaction can last from 2 hours to 24 hours, preferably from 6 to 8 hours.

The molar ratio between both reagents, namely -(-)-4-chlorobenzhydryamine and bis(2-chloroethyl)amine hydrochloride, can vary from 1:1 to 1:2, preferably 1:1.4 to 1:1.7. It was found out that by using an excess of bis(2-chloro)ethyl amine the yields can be improved.

Typically, after reaction completion, the reaction mixture is concentrated. Water and ethyl acetate are added to the residue. The pH value is adjusted with sodium hydroxide solution to alkaline prior to purification using column chromatography. The pH value of the reaction mixture prior to purification should be more than 9, preferably between 10 and 11.

The reaction is preferably carried out by reaction of -(-)-4-chlorobenzhydryamine with of bis(2-chloroethyl)amine hydrochloride in N-ethyldiisopropylamine in the presence of diethylamine at reflux temperature for several hours, isolation can be made by extraction in a solvent system consisting of water and organic solvent such as ethyl acetate, trichloromethane, dichloromethane to afford crude (-)-1-[(4-chlorophenyl)phenyl-methyl]-4-[(4-methylphenyl)sulphonyl]piperazine. The obtained product can be further purified by separation using silica gel chromatography and finally crystallized from hexane to obtain a product with HPLC purity of more than 98%.

The present process optionally comprises as step (d) the purification of the crude levocetirizine or the pharmaceutically acceptable salt thereof obtained in step (c) to attain a pure levocetirizine or a pharmaceutically acceptable salt thereof, optionally via formation of a solvate.

For example, levocetirizine dihydrochloride can be additionally recrystallized from a solvent/antisolvent mixture. As a solvent carboxylic acid or water and as an antisolvent ketone, ester or ether can be used. A preferred solvent is acetic acid or water, and a preferred antisolvent is acetone, ethyl acetate, isopropyl acetate or butyl acetate. Most preferably, as solvent acetic acid and as antisolvent acetone is used. The purity of the product obtained is greater than 99.5 %. The X-ray powder diffractogram of thus obtained levocetirizine dihydrochloride solvate with acetic acid has the following peaks at about:

| **No.** | **Position [°2Th.]** | **d-values [Å]** | **Rel. Int. [%]** |
|---|---|---|---|
| 1 | 5.9 | 14.9 | 72 |
| 2 | 11.4 | 7.77 | 81 |
| 3 | 16.8 | 5.29 | 42 |
| 4 | 17.5 | 5.06 | 41 |
| 5 | 17.8 | 4.97 | 64 |
| 6 | 21.1 | 4.22 | 59 |
| 7 | 22.5 | 3.95 | 58 |
| 8 | 26.2 | 3.40 | 100 |

It is important to control the size of the particles of levocetirizine dihydrochloride during its preparation. The average particle size of particles prepared is 5 to 200 µm, preferably between 20 and 150 µm. If unstirred, crystallization from organic solvents might also yield bigger particles, e.g. with an average diameter of above 200 µm which need to be milled or processed in any other way which reduces particle size, prior to their application in pharmaceutical formulations. When milling, particles of less than 3 µm average diameters may be produced. For this purpose air jet mills, ball mills or hammer mills are commonly used as milling equipment. However, it is not enough to control only the average size of particles, but also the particle size distribution.

Average particle size and particle size distribution is important to assure that the technological process is industrially applicable, i.e. does not cause segregation of ingredients of tab letting mixture if it is not tabletted/compressed just after preparation of tabletting mixture.

Preferably, the pure levocetirizine or the pharmaceutically acceptable salt thereof contains less than 0.2 % of the intermediate of formula II.

Another aspect of the invention is a new compound of formula IV 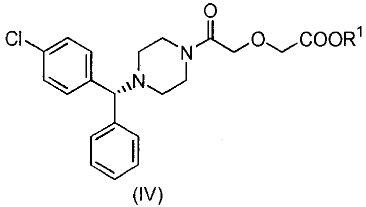 wherein R¹ is n-propyl or an organosilyl group, preferably trimethyl silyl. The ketocetirizine ester IV can be used as an intermediate for the production of levocetirizine.

The present invention is illustrated by the following Examples without being limited thereto.

### Examples

### Example 1

### Preparation of (-)-1-[(4-chlorophenyl)phenylmethyl]-4-piperazine

300 ml of N-ethyldiisopropylamine, 30 g of -(-)-4-chlorobenzhydryamine (Clemo, J. Chem. Soc. (1939) 1958-1969; Ingold, J. Chem. Soc. (1933) 1493-1505) and 42 g of bis(2-chloroethyl)amine hydrochloride were charged in a reaction vessel, stirred and heated to reflux for 3 hours. It was cooled to 60°C, 24 ml of diethylamine were added, then the mixture was heated again to reflux temperature for another 5 hours. After the reaction was completed the solvent was evaporated in vacuum, a mixture of water and ethyl acetate (1:1) was added, the pH value of the aqueous phase was adjusted to 10-11 with 30% sodium hydroxide solution. The organic phase and extract aqueous phase were separated with ethyl acetate. The combined organic phases were washed with purified water, the organic phase was decolorized with activated carbon, and the filtrate was evaporated in vacuum. The impurities were separated by use of silica gel chromatography by eluting with ethyl acetate/ethanol (7:1) and/or then with ethyl acetate/ethanol/ammonia (7:1:0.25). The eluate was collected and the solvent was evaporated in vacuum to obtain the oily residue which was further dissolved in hexane, treated with activated carbon, heated and filtered. The filtrate was cooled to 10°C for 1 hour, a precipitate was collected and dried at 40-45°C in vacuum for 5-8 hours to afford a product. HPLC (area) 98-99 %.

### Example 2

### Preparation of R-2-(2-(4-((4-chlorophenyl)(phenyl)methyl)-piperazin-2-yl-2-oxoethoxy)acetic acid

### Alternative A

2 g of R-1-((4-chlorophenyl)(phenyl)methyl)piperazine and 0.88 g of diglycolic acid anhydride were dissolved in 107 ml of acetonitrile. The reaction mixture was refluxed for 12 h. After the reaction was completed the solvent was evaporated and the obtained residue was dissolved in 20 ml water with addition of 2 ml of 1 M NaOH and then 10 ml of dichloromethane were added. The suspension was stirred for 20 minutes and the organic phase was separated. To the aqueous phase another portion of 10 ml of dichloromethane was added, the pH of the suspension was adjusted at 4.5 to 5. The aqueous phase was extracted twice again with 2 x 10 ml of dichloromethane. The organic phases were collected, dried over anhydrous sodium sulphate, filtrated and evaporated. The obtained crude product could be used in the next step without further purification.

### Alternative B

2 g of R-1-((4-chlorophenyl)(phenyl)methyl)piperazine and 0.88 g of diglycolic acid anhydride were dissolved in 10 ml of dimethylsulphoxide. To the solution 20 ml of tetrabutylammonium bromide were added. The solution was stirred for another 10 hours until the reaction was completed. The solution was diluted with 67 ml of water and 15 ml of isopropyl acetate were added. The phases were separated and the water layer was reextracted with isopropyl acetate. The organic layer was mixed with demineralised water (40 ml) and the pH of the suspension was adjusted to 10 with 2 M NaOH. The layers were separated and to the water phase 40 ml of isopropyl acetate were added. The pH of the suspension was adjusted to 3.5. The layers were separated and the water phase was reextracted twice with isopropylacetate. The organic phase was rinsed with water and evaporated to dryness. 2.5 g of product (HPLC (area) was 98.5 %)) was crystallised. The product was dried at vacuum at 50°C.

### Alternative C

2 g of R-1-((4-chlorophenyl)(phenyl)methyl)piperazine and 0.88 g of diglycolic acid anhydride were dissolved in 10 ml of dimethylsulphoxide. To the solution 20 ml of tetrabutylammonium bromide were added. The solution was stirred for about 2 hours until the reaction was completed. The solution was diluted with 67 ml of water and 15 ml of isopropyl acetate were added. The phases were separated and the water layer was reextracted with 15 ml isopropyl acetate. The collected organic layers were rinsed with demineralised water. The isopropyl acetate extract was vacuum concentrated to about 10 ml, cooled to about 0°C, stirred for another one hour. The precipitated product was filtered off, washed with 2 ml of cooled isopropyl acetate and dried at vacuum at 50°C. 2.2 g of product (HPLC (area) was 98.1 %) was obtained.

### Example 3

### Preparation of R-2-(2- (4- ((4-chlorophenyl) (phenyl)methyl) - piperazin-1-yl)-2-oxoethoxy)acetyl chloride (ketocetirizine chloride)

R-2-(2-(4-((4-chlorophenyl)(phenyl)methyl)piperazin-1-yl)-2-oxoethoxy)acetic acid (150 g) was charged into a 2 1 stirring apparatus and dissolved in tetrahydrofuran (1500 ml). The solution was cooled to 0-5°C and oxalyl dichloride (39 ml) was slowly added into solution. The solvent was removed under reduced pressure.

### Example 4

### Preparation of R-methyl 2-(2-(4-((4-chlorophenyl)(phenyl)-methyl)piperazin-1-yl)-2-oxoethoxy)acetate (methyl ester of ketocetirizine)

R-2-(2-(4-((4-chlorophenyl)(phenyl)methyl)piperazin-1-yl)-2-oxoethoxy)acetyl chloride (160 g) was charged into a 2 1 stirring apparatus and dissolved in tetrahydrofuran (1500 ml). Methanol (200 ml) was slowly added to the solution and the solvent was removed under reduced pressure.

### Example 5

### Preparation of R-methyl 2-(2-(4-((4-chlorophenyl)(phenyl)-methyl)piperazin-1-yl)-2-oxoethoxy)acetate (methyl ester of ketocetirizine)

This example is a one-pot alternative to Example 3 + Example 4. R-2-(2-(4-((4-chlorophenyl)(phenyl)methyl)piperazin-1-yl)-2-oxoethoxy)acetic acid (150 g) was charged into a 2 1 stirring apparatus and dissolved in tetrahydrofuran (1500 ml). The solution was cooled to 0-5°C and oxalyl dichloride (39 ml) was slowly added into solution. The solution was then heated to 20-25°C and methanol (200 ml) was slowly added into solution. The solvent was removed under reduced pressure.

### Example 6

### Preparation of R-methyl 2-(2-(4-((4-chlorophenyl)(phenyl)-methyl)piperazin-1-yl)ethoxy)acetate (methyl ester of levoce-tirizine)

R-methyl 2-(2-(4-((4-chlorophenyl)(phenyl)methyl)piperazin-1-yl)-2-oxoethoxy)acetate (155 g) was charged into a 2 1 stirring apparatus and dissolved in tetrahydrofuran (1500 ml). Dimethyl sulphide borane complex (83 ml; 10 M calculated on BH) was slowly added into solution and heated to reflux for additional 1 hour. The reaction mixture was cooled and methanol (200 ml) was slowly added. The solvent was removed under reduced pressure and the residue was dissolved in methanol and heated to reflux for additional 10-15 hours. The solvent was removed under reduced pressure.

### Example 7

### Preparation of R-2-(2-(4-((4-chlorophenyl)(phenyl)methyl)-piperazin-1-yl)ethoxy)acetic acid (levocetirizine)

R-methyl 2-(2-(4-((4-chlorophenyl)(phenyl)methyl)piperazin-1-yl)ethoxy)acetate (150 g) was charged into a 2 1 stirring apparatus and dissolved in 300 ml of methanol. 6 M KOH (1000 ml) was added to the solution which was then heated to 80°C for 1 hour. Methanol was removed and the solution was cooled to 20-25°C. Methyl t-butyl ether (500 ml) was added, the layers were separated and the water layer was washed with methyl t-butyl ether several times. Thereafter, dichloromethane (500 ml) was added and the pH was adjusted to 4-4.5 by addition of 6 M HCl. The product was extracted into dichloromethane, the layers were separated and the water layer was washed with dichloromethane. The combined organic layers were concentrated and the solvent was removed under reduced pressure.

### Example 8

### Preparation of R-2-(2-(4-((4-chlorophenyl)(phenyl)methyl)-piperazin-1-yl)ethoxy)acetic acid (levocetirizine)

This example is a one-pot alternative to Example 3 + Example 4 + Example 6 + Example 7. R-2-(2-(4-((4-chlorophenyl)(phenyl)-methyl)piperazin-1-yl)-2-oxoethoxy)acetic acid (150 g) was charged into a 2 1 stirring apparatus and dissolved in tetrahydrofuran (1500 ml). The solution was cooled to 0-5°C and oxalyl dichloride (39 ml) was slowly added into solution. The solution was then heated to 20-25°C and methanol (200 ml) was slowly added into solution. Thereafter, the solvent was removed under reduced pressure. The residue was dissolved in tetrahydrofuran (1500 ml) and dimethyl sulphide borane complex (83 ml; 10 M calculated on BH₃) was slowly added into solution which was then heated to reflux for additional 1 hour. The reaction mixture was then cooled and methanol (200 ml) was added. The solvent was removed under reduced pressure and the residue was dissolved in methanol (1000 ml) and heated to reflux for 10-15 hours. The solution was then concentrated and 6 M KOH (1000 ml) was added. The solution was heated to 80°C for 1 hour. Thereafter, the solution was cooled and methyl t-butyl ether (500 ml) was added. The layers were separated and the water layer was washed several times with methyl t-butyl ether. Thereafter, dichloromethane (500 ml) was added and the pH was adjusted with 6 M HCl to 4-4.5. The water layer was washed twice with dichloromethane and the combined organic layers were concentrated. The solvent was removed under reduced pressure.

### Example 9

### Preparation of propyl R-2-(2-(4-((4-chlorophenyl)phenyl-methyl)piperazin-l-yl)-2-oxoethoxy)acetate (propyl ester of ketocetirizine)

22 g of R-2-(2-(4-((4-chlorophenyl)phenylmethyl)piperazin-1-yl)-2-oxoethoxy)acetic acid (ketocetirizine) were dissolved in 110 ml of 1-propanol. During intensive stirring 4.4 ml of concentrated sulphuric acid were added and it was stirred for another one hour. After completion of the reaction 1-propanol was removed by evaporation to dryness and the oily residue was dissolved in 200 ml of toluene. 100 ml of water were added to the toluene solution and the pH of the suspension was adjusted to 8 to 9. The toluene layer was separated and the water phase was re-extracted with 200 ml of toluene. The organic layers were collected, washed with 50 ml of water and it was evaporated to dryness. Chromatographic purity: 99.7 %

### Example 10

### Preparation of levocetirizine dihydrochloride

### Alternative A

The oily residue of example 9, the propyl R-2-(2-(4-((4-chlorophenyl)phenylmethyl)piperazin-1-yl)-2-oxoethoxy)acetate, was dissolved in 220 ml of toluene, warmed up to 50°C and 11 of ml of dimethyl sulphide borane complex (10 M calculated on BH₃) were added. The reaction was stirred for another three hours and 20 ml of water were added dropwise. The reaction mixture was stirred for another half hour and 200 ml of water and 20 g of KOH were added and the suspension was stirred at reflux for another three hours.

After the hydrolysis was completed the phases were separated and the water phase was re-extracted with 200 ml of toluene and 200 ml of t-butylmethyl ether.

200 ml of methylene chloride were added to the water phase, the pH was adjusted to about 4.5 and the phases were separated. The water phase was re-extracted with same amount of methylene chloride, the organic layers were collected, washed with water and evaporated to dryness. The oily residue was dissolved in acetone and hydrogen chloride gas was introduced into solution until the pH was under 1. The obtained suspension was stirred at a temperature of about 50-60°C for about one hour, cooled to room temperature and the product was filtered off and dried to constant weight. The purity of levoce-tirizine dihydrochloride was more than 99.2 % and the content of (R)-2-(2-(4-((4-chlorophenyl)(phenyl)methyl)piperazin-1-yl)ethoxy)ethanol was less than 0.02 %.

### Alternative B

The oily residue of example 9, the propyl R-2-(2-(4-((4-chlorophenyl)phenylmethyl)piperazin-1-yl)-2-oxoethoxy)acetate, was dissolved in 220 ml of toluene, warmed up to 50°C and 11 of ml of dimethyl sulphide borane complex (10 M calculated on BH₃) were added. The reaction was stirred for another three hours and 20 ml of water were added dropwise. The reaction mixture was stirred for another half hour and 200 ml of water and 20 g of KOH were added and the suspension was stirred at reflux for another three hours.

After the hydrolysis was completed the pH of suspension was adjusted to about 7 with hydrochloric acid, phases were separated and water phase was re-extracted with toluene. After toluene extraction, pH of water suspension was adjusted to about 10 with hydrochloric acid and extraction of water phase with 200 ml of t-butylmethyl ether was performed.

200 ml of methylene chloride were added to the water phase, the pH was adjusted to about 4.5 and the phases were separated. The water phase was re-extracted with same amount of methylene chloride, the organic layers were collected, washed with water and evaporated to dryness. The oily residue was dissolved in acetone and hydrogen chloride gas was introduced into solution until the pH was below 1. The obtained suspension was stirred at a temperature of about 50-60°C for about one hour, cooled to room temperature and the product was filtered off and dried to constant weight. The purity of levocetirizine dihydrochlbride was more than 99.2 % and the content of (R)-2-(2-(4-((4-chlorophenyl)(phenyl)methyl)piperazin-1-yl)ethoxy)ethanol was less than 0.02 %.

### Example 11

### Preparation of propyl R-2-(2-(4-((4-chlorophenyl)phenyl-methyl)piperazin-1-yl)-2-oxoethoxy)acetate (propyl ester of ketocetirizine) and reduction to levocetirizine dihydrochlo-ride

22 g of R-2-(2-(4-((4-chlorophenyl)phenylmethyl)piperazin-1-yl)-2-oxoethoxy)acetic acid were dissolved in 110 ml of 1-propanol. During intensive stirring 4.4 ml of concentrated sulphuric acid were added and it was stirred for another one hour. After completion of the reaction 1-propanol was removed by evaporation to dryness and the oily residue of propyl R-2-(2-(4-((4-chlorophenyl)phenylmethyl)piperazin-1-yl)-2-oxoeth-oxy)acetate sulphate was suspended in 200 ml of toluene. The suspension was warmed up to 50°C and 11 ml of dimethyl sulphide borane complex (10 M calculated on BH₃) were added. The reaction mixture was stirred for another three hours and 20 ml of water were added dropwise. The reaction mixture was stirred for another half hour and 200 ml of water and 20 g of KOH were added and the suspension was stirred at reflux for another three hours. After the hydrolysis was completed the phases were separated and the water phase was re-extracted with 200 ml of toluene and 200 ml of t-butylmethyl ether.

200 ml of methylene dichloride were added to the water phase, the pH was adjusted to about 4.5 and the phases were separated. The water phase was re-extracted with same amount of methylene dichloride, the organic layers were collected, washed with water and evaporated to dryness. The oily residue was dissolved in acetone and hydrogen chloride gas was introduced in solution until the pH was under 1. The obtained suspension was stirred at temperature of about 50-60°C for about one hour, cooled to room temperature and the product was filtered off and dried to constant weight. The purity of levocetirizine dihydrochloride was more than 99.2 %.

### Example 12

### Preparation of propyl R-2-(2-(4-((4-chlorophenyl)phenyl-methyl)piperazin-1-yl)-2-oxoethoxy)acetate (propyl ester of ketocetirizine)

20 g of R-2-(2-(4-((4-chlorophenyl)phenylmethyl)piperazin-1-yl)-2-oxoethoxy) acetic acid were dissolved in 100 ml of 1-propanol and the suspension was cooled to -10°C. During intensive stirring HCl gas was introduced into the suspension until all starting material was dissolved. The solvent was removed by evaporation to dryness and the residue was suspended in 200 ml of toluene. 100 ml of water were added to the toluene suspension, the pH of the suspension was adjusted to from 8 to 9 and the phases were separated. The water was re-extracted with 200 ml of toluene. The collected organic phases were rinsed with water and it was evaporated to dryness.

The obtained ketocetirizine ester was reduced as described in Example 10.

### Example 13

### Preparation of propyl R-2-(2-(4-((4-chlorophenyl)phenyl-methyl)piperazin-1-yl)-2-oxoethoxy)acetate (propyl ester of ketocetirizine) and reduction to levocetirizine dihydrochlo-ride

20 g of R-2-(2-(4-((4-chlorophenyl)phenylmethyl)piperazin-1-yl)-2-oxoethoxy)acetic acid were dissolved in 100 ml of 1-propanol and the suspension was cooled to -10°C. During intensive stirring HCl gas was introduced into the suspension until all starting material was dissolved. The solvent was removed by evaporation to dryness and the residue was suspended in 200 ml of toluene.

The suspension was warmed up to 60°C and 11 ml of dimethyl sulphide borane complex (10 M calculated on BH₃) were added. The reaction was stirred for another three hours and 20 ml of water were added dropwise. The reaction mixture was stirred for another half hour and 200 ml of water and 20 g of KOH were added and the suspension was stirred at reflux for another three hours. After the hydrolysis was completed the phases were separated and the water phase was re-extracted with 200 ml of toluene and 200 ml of t-butylmethyl ether.

200 ml of methylene dichloride were added to the aqueous solution, the pH was adjusted to about 4.5 and the phases were separated. The water phase was re-extracted with the same amount of methylene dichloride, the organic layers were collected, washed with water and it was evaporated to dryness. The oily residue was dissolved in 100 ml of acetone and hydrogen chloride gas was introduced into the solution until the pH was under 1. The obtained suspension was stirred at a temperature of about 50-60°C for about one hour, cooled to room temperature and the product was filtered off and dried to constant weight. The purity of levocetirizine dihydrochloride was more than 99.3 %.

### Example 14

### Preparation of trimethylsilyl R-2-(2-(4-((4-chlorophenyl)-phenylmethyl)piperazin-1-yl)-2-oxoethoxy)acetate (silyl ester of ketocetirizine)

4,0 g of R-2-(2-(4-((4-chlorophenyl)phenylmethyl)piperazin-1-yl)-2-oxoethoxy)acetic acid were dissolved in 40 ml of tetrahydrofuran. 1.5 ml of trimethyl chlorosilane were added over a period half hour. The reaction mixture was stirred for another one hour and 3.0 ml of dimethyl sulphide borane complex (10 M calculated on BH₃) were added in one portion. The mixture was stirred at room temperature for another three hours, 100 ml of water were added and tetrahydrofuran was distilled off. 4.0 g of potassium hydroxide and 10 ml of methanol were added to the aqueous solution, it was stirred at reflux for two hours and the methanol was distilled off. 50 ml of dichloromethane were added and the ph was adjusted with 6 M HCl to 4.5. The phases were separated, the organic layers were washed with 20 ml of water and it was evaporated to dryness. The oily residue was dissolved in 20 ml of acetone and hydrogen chloride gas was introduced into the solution until the pH was under 1. The suspension was stirred at a temperature of about 50-60°C for about one hour, cooled to room temperature and the product was filtered off and dried to constant weight. Content of levocetirizine was 98.5 %.

### Example 15

### Recrystallization of levocetirizine dihydrochloride

75 g of levocetirizine dihydrochloride with 0,17 % content of compound II were dissolved in 225 ml of acetic acid at 80°C. The solution was cooled to 50°C and at this temperature 562 ml of acetone were gradually added within 1.5 hour. After addition of acetone the suspension was cooled to 0°C and stirred at this temperature for at least 1 hour. The product, levocetirizine dihydrochloride, was filtered off. The product was dried in a vacuum dryer at 50°C. There were obtained 58 g of the product which HPLC purity (area) was 99,9 %. The content of compound II was under the detection limit, which was 0.005 %.
Analysis method: HPLC; column RP18; phosphate buffer (pH 7), acetonitrile/methanol; gradient method; UV detector, 230 nm.

### Example 16

### Preparation of levocetirizine dihydrochloride solvate with acetic acid

50 g of levocetirizine dihydrochloride were suspended in 100 ml of acetic acid and heated until the substance dissolved. Then it was cooled slowly under stirring to 50°C until the suspension appeared and after that at this temperature 100 ml of acetone were added to the suspension. After the addition of acetone the suspension was cooled to a room temperature, the precipitate was filtered off and the product was washed with acetone. The product was dried in a vacuum dryer at 50°C. There were obtained 43,1 g of the product with HPLC purity (area) 99,9%.
Analysis method: HPLC; column RP18; phosphate buffer (pH 7), acetonitrile/methanol; gradient method; UV detector, 230nm.

### Example 17

### Preparation of levocetirizine dihydrochloride from levoceti-rizine dihydrochloride solvate with acetic acid

10 g of levocetirizine dihydrochloride solvate with acetic acid were suspended in 60 ml of acetone and stirred at room temperature for one hour. After one hour the suspension was filtered off and the filter cake was washed with acetone and dried in vacuum dryer. The weight of a dry product was 8,9 g. HPLC purity (area) was 99,9 %.

### Example 18

### Preparation of levocetirizine dihydrochloride via BF₃ etherate complex

5 g of R-2-(2-(4-((4-chlorophenyl)phenylmethyl)piperazin-1-yl)-2-oxoethoxy)acetic acid were dissolved in 50 ml of tetrahydrofuran (THF). 1.24 ml of BF₃ etherate were added and the reaction mixture was stirred at room temperature for another half hour. The suspension was cooled to about 0°C and 1.24 ml of oxalyl dichloride were added and the reaction mixture was warmed up to 50°C. The reaction mixture was cooled to about 0°C and 2.48 ml of dimethyl sulphide borane complex (10 M calculated on BH₃) were added dropwise. When the reaction was completed 100 ml of cold water were added to the reaction mixture and the THF was distilled off by vacuum distillation. 5 g of potassium hydroxide and 30 ml methanol were added to the aqueous solution and it was heated at reflux for another two hours. Methanol was distilled off , 50 ml of t-butylmethyl ether were added, stirred and the phases were separated. The water phase was re-extracted twice with same amount of t-butylmethyl ether. 50 ml of ethylene dichloride were added to the water phase ,the pH was adjusted to about 4.5 and the phases were separated. The water phase was re-extracted with same quantity of methylene dichloride, the organic layers were collected, washed with water and it was evaporated to dryness. The oily residue was dissolved in acetone and hydrogen chloride gas was introduced into the solution until the pH was under 1. The obtained suspension was stirred at a temperature of about 50-60°C for about one hour, cooled to room temperature and the product was filtered off and dried to constant weight. Chromatographic purity of levocetirizine dihydrochloride was 98.9 %.

### Example 19

### Synthesis of (R) -propyl 2-(2-(4-((4-chlorophenyl)phenyl-methyl)piperazin-1-yl)ethoxy)acetate dihydrochloride (propyl ester of levocetirizine dihidrochloride)

Levocetirizine dihydrochloride (2 g) was suspended in 1-propanol. (10 ml) and stirred at 50°C for 5 hours. After the reaction was completed the solvent was distilled off in vacuum. To the oily residue 20 ml of terc-butylmethyl ether was added and stirred for another two hours. The product was filtered off and dried in a vacuum dryer. 1.3 g of pure propyl ester of levocetirizine dihydrochloride was obtained.

## Claims

1. A process for the production of levocetirizine, or a pharmaceutically acceptable salt thereof, comprising the steps:
b) reaction of ketocetirizine of the formula IIIb 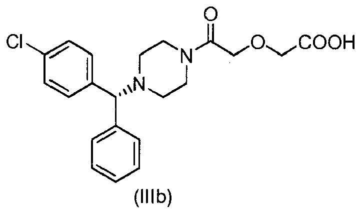 to a ketocetirizine ester of the formula IV or a salt thereof 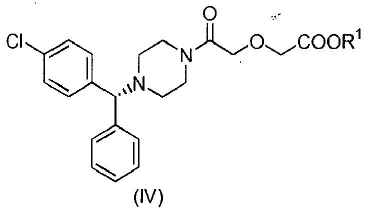 wherein R¹ is selected from alkyl groups, aryl groups, aryl substituted alkyl groups and organosilyl groups,
c) conversion of the ketocetirizine ester IV or the salt thereof to levocetirizine or a pharmaceutically acceptable salt thereof.

2. The process of claim 1, wherein R¹ is C₁ to C₆ alkyl, preferably methyl and n-propyl; or an organosilyl group, preferably trimethyl silyl.

3. The process of claims 1 or 2, wherein in step (b) the ketocetirizine of formula IIIb 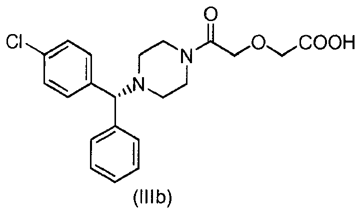 is reacted with an alcohol R¹OH, wherein R¹ is defined as in claim 1, or with an organo halosilane, preferably trimethyl chlorosilane, to form the ketocetirizine ester of the formula IV 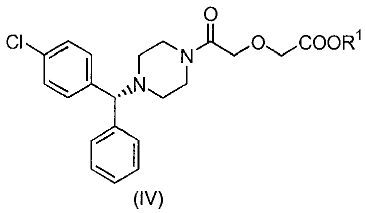 wherein R¹ is defined as in claim 1.

4. The process of any of claims 1 or 3, wherein in step (b) the ketocetirizine of formula IIIb 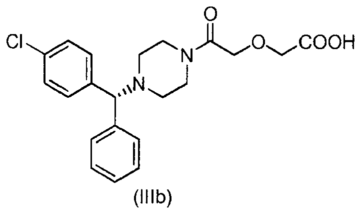 is reacted to the ketocetirizine ester of the formula IV 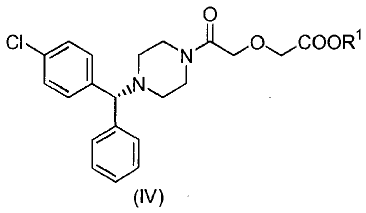 wherein R¹ is defined as in claim 1,
via the formation of ketocetirizine chloride of formula VI

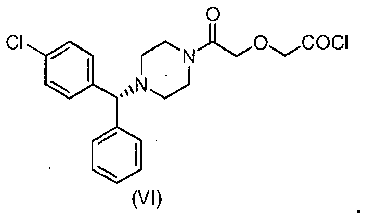

5. The process of claim 4, wherein ketocetirizine is first reacted with oxalyl dichloride to form the ketocetirizine chloride which is then reacted with an alcohol R'OH , wherin R¹ is defined as in claim 1, or with an organo silanol to form the ketocetirizine ester of the formula IV. 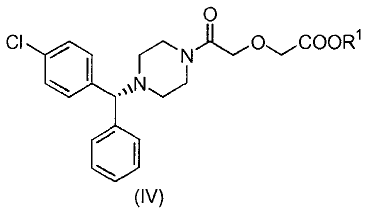 wherein R¹ is defined as in claim 1.

6. The process of claims 4 or 5, wherein the ketocetirizine chloride in not isolated prior to further reaction.

7. The process of any of the preceeding claims, wherein step (c) comprises
i) reduction of the ketocetirizine ester of the formula IV 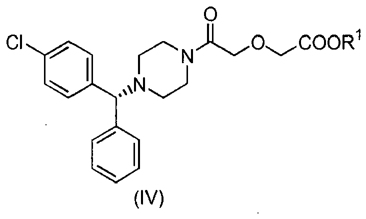 wherein R¹ is defined as in claim 1,
with a selective reducing agent to obtain an levocetirizine ester of the formula V 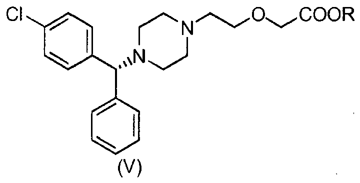 wherein R¹ is as defined above;
ii) conversion of levocetirizine ester V to levocetirizine, and
iii) optionally conversion of levocetirizine to the pharmaceutically acceptable salt thereof.

8. The process of claim 7, wherein the ester intermediate of formula V obtained in step (c)(i) is not isolated prior to step (c) (ii).

9. The process according to claims 7 or 8, wherein the selective reducing agent is selected from the group consisting of NaBH₄, optionally in the presence of a carboxylic acid or sulphonic acid; NaBH₃CN, optionally in the presence of a carboxylic acid; NaBH₃OCOR³ or NaBH(COOR³)₃ wherein R³ is methyl or trifluoromethyl; boranes; and R⁵₃OBF₄/NaBH₄ wherein R⁵ is methyl, ethyl, or propyl.

10. The process of claim 9, wherein the selective reducing agent is a borane in the form of a borane-solvent complex, wherein the solvent is selected from tetrahydrofuran (H₃B-THF), dimethyl sulphide (H₃B-SMe₂, BMS), and diethyl ether (H₃B-diethylether).

11. The process of any of the preceeding claims, wherein the ketocetirizine ester IV obtained in step (b) is not isolated prior to the conversion to levocetirizine in step (c).

12. The process of any of the preceeding claims, further comprising step (a) prior to step (b):
a) reaction of an intermediate of the formula II 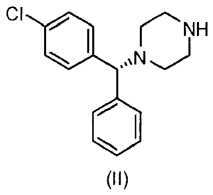 to ketocetirizine of formula IIIb.

13. The process of claim 12, wherein in step (a) the intermediate of formula II 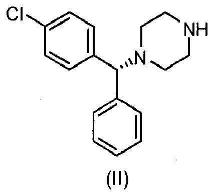 is reacted with diglycolic acid anhydride of formula 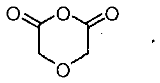 in an aprotic solvent to obtain ketocetirizine of formula IIIb.

14. The process of any of the proceeding claims, further comprising step (d) after step (c):
d) purification of the crude levocetirizine or the pharmaceutically acceptable salt thereof to obtain pure levocetirizine or a pharmaceutically acceptable salt thereof, optionally via formation of a solvate.

15. A compound of formula IV 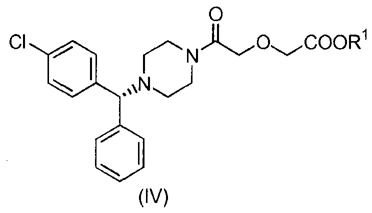 wherein R¹ is n-propyl or an organosilyl group, preferably trimethyl silyl.

16. Use of compound according to claim 15 for the production of levocetirizine.

## Patentansprüche

1. Verfahren zur Herstellung von Levocetirizin oder eines pharmazeutisch verträglichen Salzes davon, umfassend die folgenden Schritte:
b) Umsetzen von Ketocetirizin der Formel IIIb 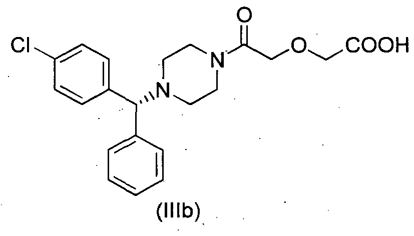 zu einem Ketocetirizinester der Formel IV oder eines Salzes davon, 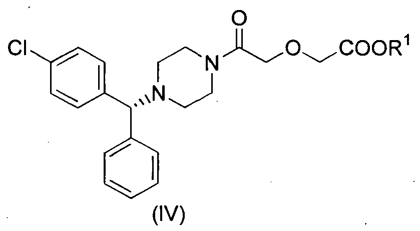 in der R¹ aus Alkylgruppen, Arylgruppen, arylsubstituierten Alkylgruppen und Organosilylgruppen ausgewählt ist,
c) Umsetzen des Ketocetirizinesters IV oder des Salzes davon zu Levocetirizin oder einem pharmazeutisch verträglichen Salzes davon.

2. Verfahren nach Anspruch 1, wobei R¹ C₁- bis C₆-Alkyl, vorzugsweise Methyl und n-Propyl, oder eine Organosilylgruppe ist, vorzugsweise Trimethylsilyl.

3. Verfahren nach Anspruch 1 oder 2, wobei in Schritt (b) das Ketocetirizin der Formel IIIb 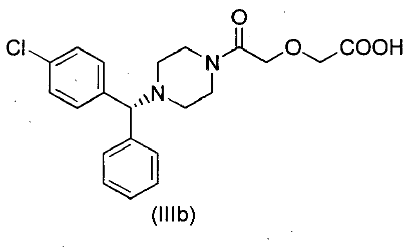 mit einem Alkohol R¹OH, wobei R¹ wie in Anspruch 1 definiert ist, oder mit einem Organohalogensilan, vorzugsweise Trimethylchlorsilan, umgesetzt wird, um den Ketocetirizinester der Formel IV zu bilden, 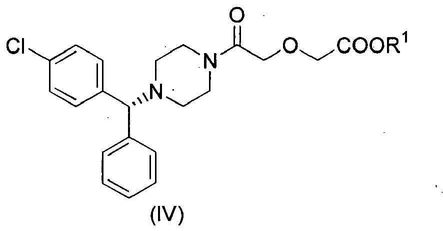 wobei R¹ wie in Anspruch 1 definiert ist.

4. Verfahren nach Anspruch 1 oder 3, wobei in Schritt (b) das Ketocetirizin der Formel IIIb 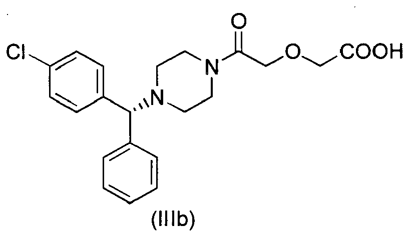 zu dem Ketocetirizinester der Formel IV umgesetzt wird, 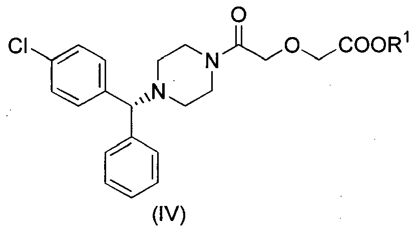 wobei R¹ wie in Anspruch 1 definiert ist,
mittels Bildung von Ketocetirizinchlorid der Formel VI

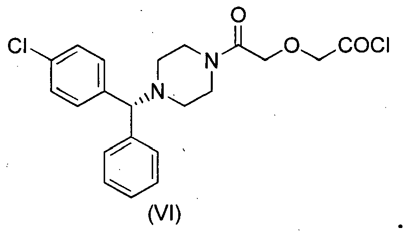

5. Verfahren nach Anspruch 4, wobei das Ketocetirizin zuerst mit Oxalyldichlorid umgesetzt wird, um das Ketocetirizinchlorid zu bilden, das dann mit einem Alkohol R¹OH, wobei R¹ wie in Anspruch 1 definiert ist, oder mit einem Organosilanol umgesetzt wird, um den Ketocetirizinester der Formel IV zu bilden, 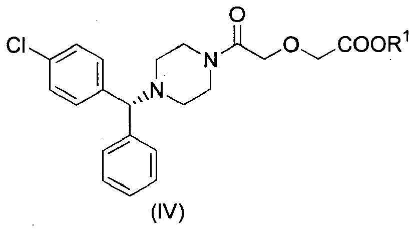 wobei R¹ wie in Anspruch 1 definiert ist.

6. Verfahren nach Anspruch 4 oder 5, wobei das Ketocetirizinchlorid vor der weiteren Umsetzung nicht isoliert wird.

7. Verfahren nach einem der vorangehenden Ansprüche, wobei Schritt (c)
i) die Reduktion des Ketocetirizinesters der Formel IV, 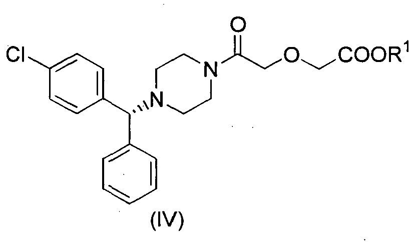 wobei R¹ wie in Anspruch 1 definiert ist,
mit einem selektiven Reduktionsmittel, um einen Levocetirizinester der Formel V zu erhalten, 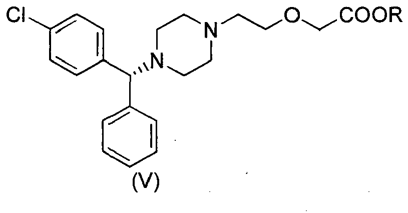 wobei R¹ wie oben definiert ist;
ii) die Umsetzung des Levocetirizinesters V zu Levocetirizin und
iii) gegebenenfalls die Umsetzung von Levocetirizin zu einem pharmazeutisch verträglichen Salz davon
umfasst.

8. Verfahren nach Anspruch 7, wobei das in Schritt (c)(i) erhaltene Esterintermediat der Formel V vor Schritt (c) (ii) nicht isoliert wird.

9. Verfahren nach Anspruch 7 oder 8, wobei das selektive Reduktionsmittel aus der aus NaBH₄, gegebenenfalls in Anwesenheit einer Carbonsäure oder Sulfonsäure, NaBH₃CN, gegebenenfalls in Anwesenheit einer Carbonsäure, NaBH₃OCOR³ oder NaBH(COOR³)₃, wobei R³ Methyl oder Trifluormethyl ist, Boranen und R⁵₃OBF₄/NaBH₄ bestehenden Gruppe ausgewählt ist, wobei R⁵ Methyl, Ehtyl oder Propyl ist.

10. Verfahren nach Anspruch 9, wobei das selektive Reduktionsmittel ein Boran in der Form eines Boran-Lösungsmittel-Komplexes ist, wobei das Lösungsmittel aus Tetrahydrofuran (H₃B-THF), Dimethylsulfid (H₃B-SMe₂, BMS) und Diethylether (H₃B-Diethylether) ausgewählt ist.

11. Verfahren nach einem der vorangehenden Ansprüche, wobei der in Schritt (b) erhaltene Ketocetirizinester IV vor der Umsetzung zu Levocetirizin in Schritt (c) nicht isoliert wird.

12. Verfahren nach einem der vorangehenden Ansprüche, das weiter den Schritt (a) vor dem Schritt (b) umfasst:
a) Umsetzen eines Intermediats der Formel II 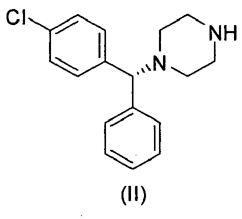 zu Ketocetirizin der Formel IIIb.

13. Verfahren nach Anspruch 12, bei dem in Schritt (a) das Intermediat der Formel II 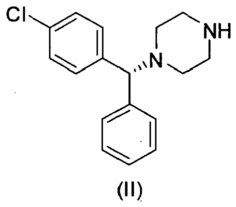 mit Diglycolsäureanhydrid der Formel 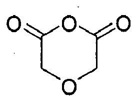 in einem aprotischen Lösungsmittel umgesetzt wird, um Ketocetirizin der Formel IIIb zu erhalten.

14. Verfahren nach einem der vorangehenden Ansprüche, das weiter Schritt (d) nach Schritt (c) umfasst:
d) Reinigen des rohen Levocetirizins oder des pharmazeutisch verträglichen Salzes davon, um reines Levocetirizin oder ein pharmazeutisch verträgliches Salz davon zu erhalten, gegebenenfalls mittels Bildung eines Solvats.

15. Verbindung gemäß Formel IV, 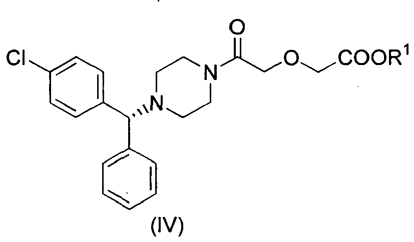 in der R¹ n-Propyl oder eine Organosilylgruppe, vorzugsweise Trimethylsilyl, ist.

16. Verwendung einer Verbindung gemäß Anspruch 15 zur Herstellung von Levocetirizin.

## Revendications

1. Procédé de préparation de la lévocétirizine ou d'un sel pharmacologiquement admissible de ce composé, lequel procédé comporte les étapes suivantes :
b) faire réagir la kétocétirizine, de formule (IIIb) : 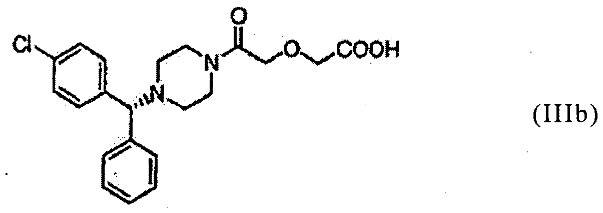 de manière à la convertir en un ester de kétocétirizine, de formule (IV), ou en un sel d'un tel ester : 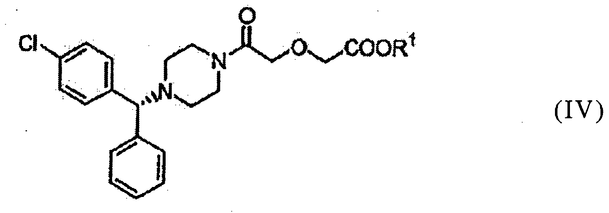 dans laquelle R¹ représente une entité choisie parmi les groupes alkyle, les groupes aryle, les groupes alkyle à substituant(s) aryle, et les groupes organo-silyle ;
c) et convertir cet ester de kétocétirizine (IV) ou son sel en lévocétirizine ou en un sel pharmacologiquement admissible de ce composé.

2. Procédé conforme à la revendication 1, dans lequel R¹ représente un groupe alkyle en C₁-C₆, de préférence un groupe méthyle ou n-propyle, ou un groupe organo-silyle, de préférence un groupe triméthyl-silyle.

3. Procédé conforme à la revendication 1 ou 2, dans lequel, dans l'étape (b), on fait réagir de la kétocétirizine, de formule (IIIb) : 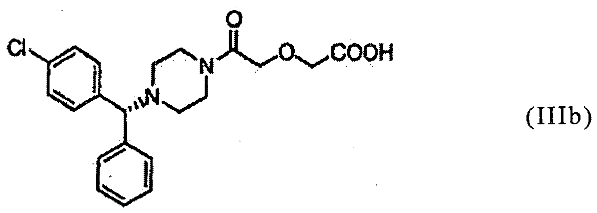 avec un alcool de formule R¹OH, dans laquelle R¹ a la signification indiquée dans la revendication 1, ou avec un organo-halogéno-silane, de préférence du triméthyl-chloro-silane, de manière à ce qu'il se forme un ester de kétocétirizine, de formule (IV) : 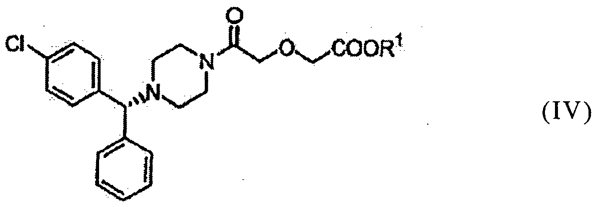 dans laquelle R¹ a la signification indiquée dans la revendication 1.

4. Procédé conforme à la revendication 1 ou 3, dans lequel, dans l'étape (b), on fait réagir de la kétocétirizine, de formule (IIIb) : 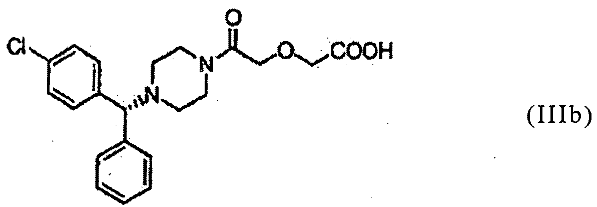 de manière à la convertir en un ester de kétocétirizine, de formule (IV) : 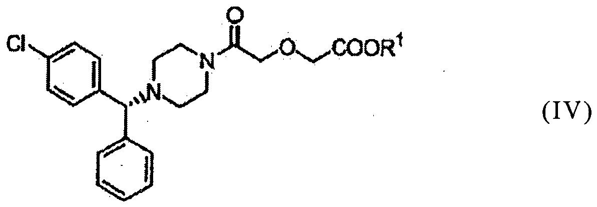 dans laquelle R¹ a la signification indiquée dans la revendication 1,
via la formation du chlorure de kétocétirizine, de formule (VI) :

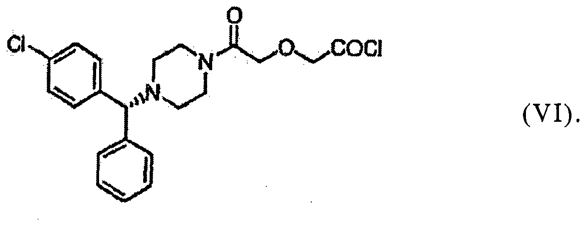

5. Procédé conforme à la revendication 4, dans lequel on fait d'abord réagir la kétocétirizine avec du dichlorure d'oxalyle, de manière à ce qu'il se forme du chlorure de kétocétirizine, que l'on fait ensuite réagir avec un alcool de formule R¹OH, dans laquelle R¹ a la signification indiquée dans la revendication 1, ou avec un organo-silanol, de manière à ce qu'il se forme un ester de kétocétirizine, de formule (IV) : 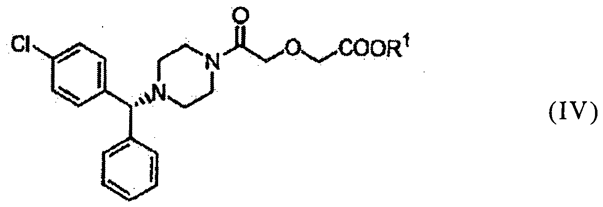 dans laquelle R¹ a la signification indiquée dans la revendication 1.

6. Procédé conforme à la revendication 4 ou 5, dans lequel on n'isole pas le chlorure de kétocétirizine, avant de le faire ultérieurement réagir.

7. Procédé conforme à l'une des revendications précédentes, dans lequel l'étape (c) comporte :
i) le fait de réduire l'ester de kétocétirizine de formule IV 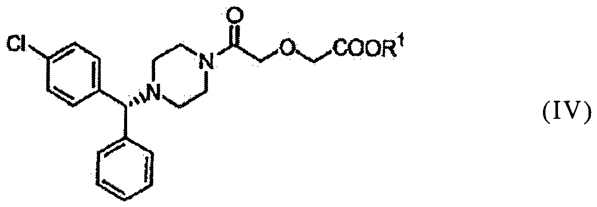 dans laquelle R¹ a la signification indiquée dans la revendication 1, au moyen d'un agent réducteur sélectif, de manière à obtenir un ester de lévocétirizine, de formule (V) : 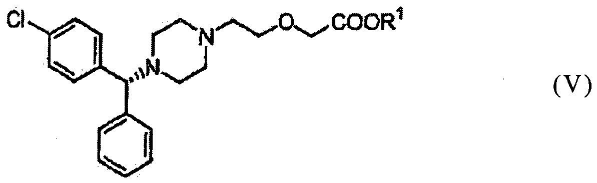 dans laquelle R¹ a la signification indiquée plus haut ;
ii) et le fait de convertir l'ester de lévocétirizine de formule V en lévocétirizine ;
iii) et en option, le fait de convertir la lévocétirizine en l'un de ses sels pharmacologiquement admissibles.

8. Procédé conforme à la revendication 7, dans lequel, avant l'étape (c-ii), on n'isole pas l'ester intermédiaire de formule (V) obtenu dans l'étape (c-i).

9. Procédé conforme à la revendication 7 ou 8, dans lequel l'agent réducteur sélectif est choisi dans l'ensemble constitué par les suivants :
- du borohydrure de sodium NaBH₄, en présence, en option, d'un acide carboxylique ou d'un acide sulfonique,
- du borocyanohydrure de sodium NaBH₃CN, en présence, en option, d'un acide carboxylique,
- les composés de formule NaBH₃OCOR³ ou NaBH(COOR³)₃ dans laquelle R³ représente un groupe méthyle ou trifluoro-méthyle,
- les boranes,
- et les composés de formule R⁵₃OBF₄/NaBH₄ dans laquelle R⁵ représente un groupe méthyle, éthyle ou propyle.

10. Procédé conforme à la revendication 9, dans lequel l'agent réducteur sélectif est un borane sous forme d'un complexe solvant-borane pour lequel le solvant est choisi parmi le tétrahydrofurane (complexe H₃B-THF), le sulfure de diméthyle (complexe H₃B-SMe₂, BMS), et l'éther diéthylique (complexe H₃B-OEt₂).

11. Procédé conforme à l'une des revendications précédentes, dans lequel on n'isole pas l'ester de kétocétirizine de formule (IV), obtenu dans l'étape (b), avant de le convertir en lévocétirizine dans l'étape (c).

12. Procédé conforme à l'une des revendications précédentes, qui comporte en outre, avant l'étape (b), l'étape (a) suivante :
a) faire réagir le produit intermédiaire de formule (II) : 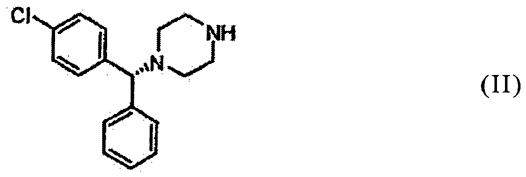 de manière à le convertir en de la kétocétirizine, de formule (IIIb).

13. Procédé conforme à la revendication 12, dans lequel, dans l'étape (a), on fait réagir le produit intermédiaire de formule (II) : 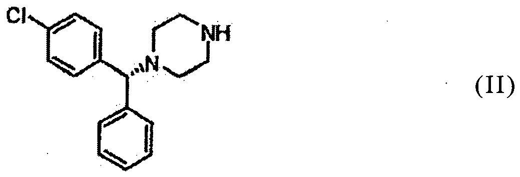 avec de l'anhydride d'acide diglycolique, de formule 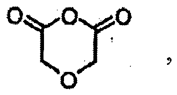 dans un solvant aprotique, de manière à obtenir de la kétocétirizine, de formule (IIIb).

14. Procédé conforme à l'une des revendications précédentes, qui comporte en outre, après l'étape (c), l'étape (d) suivante :
d) purifier le produit brut, la lévocétirizine ou son sel pharmacologiquement admissible, pour obtenir à l'état pur la lévocétirizine ou son sel pharmacologiquement admissible, en option via la formation d'un solvat.

15. Composé de formule (IV) : 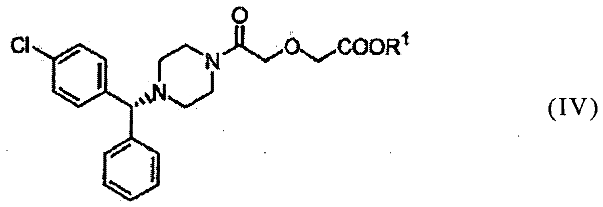 dans laquelle R¹ représente un groupe n-propyle, ou un groupe organo-silyle, de préférence un groupe triméthyl-silyle.

16. Utilisation d'un composé conforme à la revendication 15 pour la préparation de la lévocétirizine.
